# EUROPEAN PATENT SPECIFICATION

(11) **EP 1 721 616 B1**
(45) Date of publication and mention of the grant of the patent: **01.04.2009**
(21) Application number: 05709804.8
(22) Date of filing: 07.02.2005
(51) Int. Cl.: A61K 38/01, A61P 3/02

(54) **PROTEIN HYDROLYSATE FOR TREATING FATIGUE**
PROTEINHYDROLYSAT ZUR BEHANDLUNG VON MÜDIGKEIT
HYDROLYSAT DE PROTEINES POUR LE TRAITEMENT DE LA FATIGUE

(30) Priority: 24.02.2004 JP 2004048417
(43) Date of publication of application: 15.11.2006
(73) Proprietor: Ajinomoto Co., Inc., Tokyo 104-8315 (JP)
(72) Inventor: KODERA, Tomohiro c/o Ajinomoto Co., Inc., Kawasaki-shi, Kanagawa 2108681 (JP); NIO, Noriki c/o Ajinomoto Co., Inc., Chuo-ku, Tokyo 1048315 (JP); MURAKAMI, Hitoshi c/o Ajinomoto Co., Inc., Kawasaki-shi, Kanagawa 2108681 (JP)
(74) Representative: Denison, Christopher Marcus
(86) International application number: PCT/JP2005/001752
(87) International publication number: WO 2005/079826

(56) References cited:
- JP-A- 1 269 456
- JP-A- 5 505 524
- JP-A- 8 000 264
- JP-A- 63 287 462
- JP-A- 2000 083 695
- US-A- 5 866 357

### SEQUENCE LISTING

<110> AJINOMOTO Co., Inc.
<120> Restorative composition and food
<130> PH-2322PCT
<150> JP2004-048417
   <151> 2004-2-24
<160> 3
<170> PatentIn version 3.1
<210> 1
   <211> 247
   <212> PRT
   <213> Glycine max
<400> 1
   Asp Lys Leu Pro Glu Ser Val Asp Trp Arg Lys Glu Gly Ala Val Pro
   1               5                   10                  15
   Pro Val Lys Asp Gln Gly Gly Cys Gly Ser Cys Trp Ala Phe Ser Ala
               20                  25                  30
   Ile Gly Ala Val Glu Gly Ile Asn Lys Ile Val Thr Gly Glu Leu Ile
   35 40 45
   Ser Leu Ser Glu Gln Glu Leu Val Asp Cys Asp Thr Gly Tyr Asn Glu
   50 55 60
   Gly Cys Asn Gly Gly Leu Met Asp Tyr Ala Phe Glu Phe Ile Ile Asn
   65 70 75 80
   Asn Gly Gly Ile Asp Ser Glu Glu Asp Tyr Pro Tyr Arg Gly Val Asp
   85 90 95
   Gly Arg Cys Asp Thr Tyr Arg Lys Asn Ala Lys Val Val Ser Ile Asp
   100 105 110
   Asp Tyr Glu Asp Val Pro Ala Tyr Asp Glu Leu Ala Leu Lys Lys Ala
   115 120 125
   Val Ala Asn Gln Pro Val Ser Val Ala Ile Glu Gly Gly Gly Arg Glu
   130 135 140
   Phe Gln Leu Tyr Val Ser Gly Val Phe Thr Gly Arg Cys Gly Thr Ala
   145 150 155 160
   Leu Asp His Gly Val Val Ala Val Gly Tyr Gly Thr Ala Asn Gly His
   165 170 175
   Asp Tyr Trp Ile Val Arg Asn Ser Trp Gly Pro Ser Trp Gly Glu Asp
   180 185 190
   Gly Tyr Ile Arg Leu Glu Arg Asn Leu Ala Asn Ser Arg Ser Gly Lys
   195 200 205
   Cys Gly Ile Ala Ile Glu Pro Ser Tyr Pro Leu Lys Asn Gly Pro Asn
   210 215 220
   Pro Pro Asn Pro Gly Pro Ser Pro Pro Ser Pro Val Lys Pro Pro Asn
   225 230 235 240
   Val Cys Asp Asn Tyr Tyr Ser
   245
<210> 2
   <211> 247
   <212> PRT
   <213> Glycine max
<400> 2
   Asp Lys Leu Pro Asp Ser Val Asp Trp Arg Lys Glu Gly Ala Val Pro
   1 5 10 15
   Pro Val Lys Asp Gln Gly Gly Cys Gly Ser Cys Trp Ala Phe Ser Ala
   20 25 30
   Ile Gly Ala Val Glu Gly Ile Asn Lys Ile Val Thr Gly Glu Leu Ile
   35 40 45
   Ser Leu Ser Glu Gln Glu Leu Val Asp Cys Asp Thr Gly Tyr Asn Gln
   50 55 60
   Gly Cys Asn Gly Gly Leu Met Asp Tyr Ala Phe Glu Phe Ile Ile Asn
   65 70 75 80
   Asn Gly Gly Ile Asp Ser Asp Glu Asp Tyr Pro Tyr Arg Gly Val Asp
   85 90 95
   Gly Arg Cys Asp Thr Tyr Arg Lys Asn Ala Lys Val Val Ser Ile Asp
   100 105 110
   Asp Tyr Glu Asp Val Pro Ala Tyr Asp Glu Leu Ala Leu Lys Lys Ala
   115 120 125
   Val Ala Asn Gln Pro Val Ser Val Ala Ile Glu Gly Gly Gly Arg Glu
   130 135 140
   Phe Gln Leu Tyr Val Ser Gly Val Phe Thr Gly Arg Cys Gly Thr Ala
   145 150 155 160
   Leu Asp His Gly Val Val Ala Val Gly Tyr Gly Thr Ala Lys Gly His
   165 170 175
   Asp Tyr Trp Ile Val Arg Asn Ser Trp Gly Ser Ser Trp Gly Glu Asp
   180 185 190
   Gly Tyr Ile Arg Leu Glu Arg Asn Leu Ala Asn Ser Arg Ser Gly Lys
   195 200 205
   Cys Gly Ile Ala Ile Glu Pro Ser Tyr Pro Leu Lys Asn Gly Pro Asn
   210 215 220
   Pro Pro Asn Pro Gly Pro Ser Pro Pro Ser Pro Val Lys Pro Pro Asn
   225 230 235 240
   Val Cys Asp Asn Tyr Tyr Ser
   245
<210> 3
   <211> 222
   <212> PRT
   <213> Bacillus licheniformis
<400> 3
   Ser Val Ile Gly Ser Asp Asp Arg Thr Arg Val Thr Asn Thr Thr Ala
   1 5 10 15
   Tyr Pro Tyr Arg Ala Ile Val His Ile Ser Ser Ser Ile Gly Ser Cys
   20 25 30
   Thr Gly Trp Met Ile Gly Pro Lys Thr Val Ala Thr Ala Gly His Cys
   35 40 45
   Ile Tyr Asp Thr Ser Ser Gly Ser Phe Ala Gly Thr Ala Thr Val Ser
   50 55 60
   Pro Gly Arg Asn Gly Thr Ser Tyr Pro Tyr Gly Ser Val Lys Ser Thr
   65 70 75 80
   Arg Tyr Phe Ile Pro Ser Gly Trp Arg Ser Gly Asn Thr Asn Tyr Asp
   85 90 95
   Tyr Gly Ala Ile Glu Leu Ser Glu Pro Ile Gly Asn Thr Val Gly Tyr
   100 105 110
   Phe Gly Tyr Ser Tyr Thr Thr Ser Ser Leu Val Gly Thr Thr Val Thr
   115 120 125
   Ile Ser Gly Tyr Pro Gly Asp Lys Thr Ala Gly Thr Gln Trp Gln His
   130 135 140
   Ser Gly Pro Ile Ala Ile Ser Glu Thr Tyr Lys Leu Gln Tyr Ala Met
   145 150 155 160
   Asp Thr Tyr Gly Gly Gln Ser Gly Ser Pro Val Phe Glu Gln Ser Ser
   165 170 175
   Ser Arg Thr Asn Cys Ser Gly Pro Cys Ser Leu Ala Val His Thr Asn
   180 185 190
   Gly Val Tyr Gly Gly Ser Ser Tyr Asn Arg Gly Thr Arg Ile Thr Lys
   195 200 205
   Glu Val Phe Asp Asn Leu Thr Asn Trp Lys Asn Ser Ala Gln
   210 215 220

## Description

### Technical Field

The present invention relates to a soothing agent and a food for treating fatigue, which are used by individuals who feel tired in daily life, feel exhausted after hard or hours-long exercise or labor, or have unidentified complaints upon awakening.

### Background Art

Heretofore, revitalizing beverages that have been widely used as soothing agents by individuals in daily life mainly consisted of various vitamins, caffeine, taurine, and the like. However, for example, vitamins and taurine have not yet been confirmed to have obvious soothing effects. Moreover, since caffeine, which has been confirmed to have soothing effects, acts on the central nervous system, it does not essentially resolve causes of fatigue.

In addition, even though Korean ginseng, royal jelly, and propolis protein have been confirmed to have soothing effects, the action sites and action mechanisms thereof, for example, are unclear. Further, products thereof are significantly problematic in terms of sensory perceptions such as taste and smell. Thus, it is difficult to use such products in forms different from those used for pharmaceuticals. Therefore, it is considered that it would be difficult to widely use such products in a daily diet.

Moreover, it has been reported that dipeptides such as anserine and carnosine have soothing effects. However, since such enzymatic hydrolysates are very bitter, they are still problematic in terms of sensory perceptions.

Thus, a substance that does not cause problems in terms of sensory perceptions and has soothing effects has been awaited.
[Patent Document 1] JP Patent Publication (Kohyo) No. 5-505524 A (1993)
[Patent Document 2] JP Patent Publication (Kokai) No. 8-264 A (1996)
[Patent Document 3] JP Patent Publication (Kokai) No. 9-121870 A (1997)
[Patent Document 4] JP Patent Publication (Kokai) No. 2000-83695 A
[Patent Document 5] US Patent No. 4266031
[Non-Patent Document 1] "Molecular Cloning: A Laboratory Manual," Cold Spring Harbor, 1982, p. 4

### Disclosure of the Invention

It is an objective of the present invention to provide a soothing agent and a food for treating fatigue whereby: physical strength can be quickly restored from exhaustion (a state with lowered spontaneous motility), such as after exercise or exhausting labor or upon awakening in the early morning; physical strength decline can be prevented; and physical strength can be maintained.

As a result of intensive studies, inventors of the present invention have found that protein hydrolysates that can be obtained by degrading protein via germinated soybean cotyledon-derived thiol protease and/or microorganism-derived serine protease have excellent soothing effects and revitalizing effects. This has led to the completion of the present invention.

Specifically, the present invention is defined in the claims.

The invention is directed to Protein hydrolysates, hydrolysed by germinated soybean cotyledon-derived thiol protease and/or micro-organism derived serine protease, for use in a method of treating fatigue. Use of protein hydrolysates, hydrolysed by germinated soybean cotyledon-derived thiol protease and/or micro-organism derived serine protease, in the manufacture of a medicament for treating fatigue.

In accordance with the present application, a soothing agent and a food for treating fatigue, which have excellent soothing effects and revitalizing effects, are described. In addition, the soothing agent and the food for treating fatigue can be readily ingested without problems such as bitterness in terms of sensory perceptions.

### Brief Description of the Drawings

Fig. 1 shows the results of Example
Fig. 2 shows the results of Example 2 (changes in spontaneous motility).
Fig. 3 shows the results of Example 3 (changes in spontaneous motility).
Fig. 4 shows the results of Example 4 (changes in spontaneous motility).
Fig. 5 shows the results of Example 5 (changes in spontaneous motility).

### Best Mode for Carrying Out the Invention

Hereafter, the present invention will be described in detail. The protein hydrolysates used in the present invention are obtained by degrading substrate protein via germinated soybean cotyledon-derived thiol protease and/or microorganism-derived serine protease.

The thiol protease derived from germinating soybean cotyledon (hereafter sometimes referred to as "D3") used in the present invention has been known to the public. The protease is derived from germinated soybean cotyledons and it can degrade soybean seed storage protein into amino acids or even into low-molecular-weight peptides. The protease has been known to have the following properties:
(a) optimum pH: approximately pH 3 to 7;
(b) optimum temperature: approximately 30°C to 50°C;
(c) molecular weight (SDS-PAGE): approximately 26 to 30 KD;
(d) inhibitor: inhibited by trans-epoxysuccinyl-L-leucylamide(4-guanidino)-butane; and
(e) activator: activated by 2-mercaptoethanol, cysteine, and reduced glutathione.

D3 can be separated and purified from germinated soybean cotyledons in accordance with a known method such as that described in JP Patent Publication (Kokai) No. 8-264 A (1996).

In addition, as D3 used in the present invention, not only D3 that can be directly obtained from germinated soybean cotyledons but also recombinant D3 that can be produced by a transformant can be used. A method for producing D3 using a transformant has been known to the public. For instance, a transformant that produces D3 can be obtained by incorporating D3-encoding DNA into an adequate expression vector and introducing the expression vector into microorganisms such as *Escherichia coli* and yeast or cells. Such method for producing recombinant D3 is described in detail in, for example, JP Patent Publication (Kokai) No. 9-121870 A (1997): "DNA capable of coding new thiol protease and production of the same thiol protease using the DNA."

There are two types of D3 isozymes (D3-α and D3-β) and both can be used. Amino acid sequences of D3-α D3-β are set forth in SEQ ID NO:1 and SEQ ID NO:2, respectively.

As D3 that can be used in the present invention, not only D3 having the amino acid sequence set forth in SEQ ID NO:1 or 2 but also a derivative thereof can be used. Herein, it is understood that the term "derivative" of D3 indicates a proteolytic enzyme induced from natural enzyme D3, provided that proteolytic activity of the enzyme is not damaged by addition of one or more amino acids to the C-terminal and/or the N-terminal of a natural amino acid sequence of natural protein D3, substitution of one or more amino acids at one or more different sites of the natural amino acid sequence, deletion of one or more amino acids at either one of or both ends of the natural protein or at one or more sites of the amino acid sequence, or insertion of one or more amino acids at one or more sites of the natural amino acid sequence.

Next, microorganism-derived serine protease (hereafter sometimes referred to as "GSP") that can be used in the present invention will be described below.

The serine protease is a known protease that specifically cleaves the C-terminal of glutamic acid (Glu) residue and the C-terminal of aspartic acid (Asp) residue, and it has been known to have the following properties:
(a) optimum pH: approximately pH 6.5 to 10;
(b) optimum temperature: approximately 40°C to 65°C;
(c) molecular weight (SDS-PAGE): approximately 22 to 28 KD;
(d) inhibitor: inhibited by diisopropylphosphofluoridate but not by phenylmethanesulfonyl fluoride; and
(e) activator: calcium ion.

Examples of microorganisms that produce serine protease as described above include microorganisms belonging to the genera *Bacillus, Streptomyces, Staphylococcus, Actinomyces,* and the like. Specific examples thereof include *Bacillus licheniformis, Bacillus subtilis, Streptomyces thermovulgaris, Streptomyces griseus,* and *Staphylococcus aureus.* The GSP used for the present invention can be isolated and purified from culture products of microorganisms as described above in accordance with known methods such as those described in US Patent No. 4,266,031 and JP Patent Publication (Kohyo) No. 5-505524 A (1993).

In addition, the GSP used for the present invention, which is derived from a microorganism belonging to the genus *Bacillus,* may be obtained from a mutant of a microorganism belonging to the genus *Bacillus,* such as a mutant obtained via deactivation of a gene encoding subtilisin A by a general mutagenesis method that comprises the use of a mutagen (e.g., nitrosoguanidine) in a method disclosed in the aforementioned US Patent No. 4,266,031, for example. Further, the GSP used for the present invention may be GSP obtained via gene recombination. Specifically such GSP is obtained by: isolating a GSP-encoding DNA sequence from cDNA or genome library of, for example, *Bacillus licheniformis,* which is a microorganism producing the enzyme; inserting the DNA sequence into an adequate expression vector; transforming an adequate host microorganism using the vector; allowing the host to proliferate under conditions that facilitate production of the enzyme; and recovering GSP from the culture product. These steps can be carried out via a standard method (see T. Maniatis et al., Molecular Cloning: A Laboratory Manual, Cold Spring Harbor, 1982). In addition, the amino acid sequence of GSP is set forth in SEQ ID NO:3.

As GSP that can be used for the present invention, not only GSP having the amino acid sequence set forth in SEQ ID NO:3 but also a derivative thereof can be used. Herein, it is understood that the term "derivative" of GSP indicates a proteolytic enzyme induced from natural enzyme GSP, provided that proteolytic activity of the enzyme is not damaged by addition of one or more amino acids to the C-terminal and/or the N-terminal of a natural amino acid sequence of natural protein GSP, substitution of one or more amino acids at one or more different sites of the natural amino acid sequence, deletion of one or more amino acids at either one of or both ends of the natural protein or at one or more sites of the amino acid sequence, or insertion of one or more amino acids at one or more sites of the natural amino acid sequence.

Even in a case where GSP is prepared using a mutant from a mutant line, in which other proteases have been inactivated, such as a *Bacillus licheniformis* strain in which a gene encoding subtilisin A has been substantially inactivated, it is preferable to obtain a desired protease fraction from a culture broth. Examples of fractionation methods may be any fractionation methods using ammonium sulfate precipitation, membrane treatment, ion-exchange resin, gel filtration chromatography, or the like. The presence of an enzyme that is not protease does not influence protein hydrolysate properties.

The protein material used for the present invention is not particularly limited as long as it is a substance or ingredient that contains protein components. Preferred examples thereof contain protein components such as: animal proteins including whey protein, casein, meat proteins, fish proteins, erythrocyte, albumen, collagen, and gelatin; plant proteins including soy protein, wheat gluten, zein, rapeseed protein, alfalfa protein, pea protein, legume protein, cotton protein, potato protein, rice protein, and sesame protein; and grain protein. In the present invention, as protein materials, casein, wheat protein, and dried bonito flakes are particularly preferable.

The protein hydrolysates used for the present invention can be prepared in the following manner.

An example of preparation of a protein hydrolysate using germinated soybean cotyledon-derived protease D3 will be described below. First, a protein aqueous solution at a concentration of about 1% to 10% by weight is prepared. The solution is heated according to need (e.g., heating at 121°C for 15 minutes). Then, the pH of the solution is adjusted to pH 3 to 6 and preferably pH 3.5 to 5 using hydrochloric acid, sulfuric acid, or the like. To the protein solution, protease D3 is added in an amount of 0.05 to 2 g and preferably 0.1 to 1 g relative to 100 g of protein. Subsequently, the protein solution to which D3 has been added is subjected to enzymatic reaction at 30°C to 50°C (preferably 35°C to 45°C) for 1 to 100 hours and preferably 24 to 48 hours. After termination of the reaction, the reaction solution is heated (e.g. heat treating at 80°C for 20 minutes), for enzyme deactivation. Then, the resulting solution is subjected to centrifugation so that a supernatant fraction containing protein hydrolysates is obtained. The supernatant thereof is neutralized using NaOH such that the pH thereof becomes roughly neutral. Thereafter, the resultant is freeze dried. Thus, protein hydrolysates can be obtained.

Next, an example of preparation of a GSP-degraded product will be described below.

While preparing protein hydrolysates by serine protease GSP, the pH of a protein aqueous solution tends to decrease from neutral or weak alkaline to a lower pH level along with the progression of reaction. Thus, during degradation reaction of protein material, it is preferable to maintain the pH at a constant level. The pH can be maintained by titrating the protein aqueous solution with bases such as NaOH, KOH, Ca (OH)₂, and NH₃. Also, it is convenient to automatically perform pH monitoring and titration using a pH-stat.

In an example of preparing protein hydrolysates using serine protease GSP, first, a protein aqueous solution at a concentration of about 1% to 10% by weight is prepared. Then, the solution is heated according to need (e.g., heating at 121°C for 15 minutes). Then, the pH of the solution is adjusted to approximately pH 7 to 11 and preferably pH 7 to 9 using bases (e.g., NaOH, KOH, and NaHCO₃). To the protein solution, protease GSP is added in an amount of 0.05 to 2 g and preferably 0.1 to 1 g relative to 100 g of protein such that sufficient hydrolysis takes place. Subsequently, the protein solution to which GSP has been added is subjected to enzymatic reaction at 30°C to 50°C (preferably 40°C to 50°C) for 1 to 48 hours and preferably 4 to 8 hours. After termination of the reaction, the reaction solution is heated (e.g., heat treating at 80°C for 20 minutes) for enzyme deactivation. Then, the resulting solution is subjected to centrifugation so that a supernatant fraction containing protein hydrolysates is obtained. The supernatant thereof is neutralized such that the pH thereof becomes roughly neutral. Thereafter, the resultant is freeze dried. Thus, protein hydrolysates can be obtained.

As described above, protein hydrolysate by D3 and/or GSP such that a mixture of protein hydrolysates is obtained. An enzymatic degradation reaction is carried out in a manner such that the molecular weights of enzymatic hydrolysates are between 150 and 20,000 and preferably between 500 and 5,000. In addition, it is preferable to carry out an enzymatic degradation reaction in a manner such that low-molecular-weight peptide components (molecular weight: between 150 and 20,000 and preferably between 500 and 5,000) in the mixture of protein hydrolysates obtained via enzymatic degradation account for 60% by weight of the total obtained peptide mixture.

The soothing agent or the revitalizing agent described herein (hereafter sometimes referred to as a nutritional agent of the present invention) contains protein hydrolysates obtained as described above. It may be formulated into capsules, powders, tablets, granules, fine grains, syrups, solutions, suspensions, agents for intrarectal administration, injections, infusions, and the like. These formulations can be readily produced in accordance with general formulation methods in the field of pharmaceutical technology.

Moreover, the aforementioned protein hydrolysates may be contained in a food for treating fatigue or a revitalizing food. Forms of such food are not particularly limited. Examples thereof include powders, solid foods, liquid foods, beverages, and jellies. In addition to these examples, a nutritional supplement, such as a so-called "supplement," may be included.

The nutritional agent and the food of the present invention may contain other nutritional components, medicinal components, additives generally used in the fields of medicine manufacturing and food industry, and the like, in addition to protein hydrolysates. Examples of such additional components and additives include amino acids, vitamins, carbohydrates, lipids, proteins, vitamins, minerals, caffeine, and crude drugs. Examples of such additives include dyes, diluents, flavorings, antiseptics, excipients, disintegrants, lubricants, binders, surfactants, plasticizers, preservatives, and antioxidants.

The doses (intakes) of the nutritional agent and the food of the present invention will be described below. As a result of an experiment in which protein hydrolysates (peptides formed via enzymatic degradation) were administered to mice, the present invention was found to have effects of soothing fatigue under the condition that the dose thereof was more than 1 g (dry weight) /kg. Such dose for animals is converted to a dose thereof for humans. Given that daily protein intake for mice is about 0.9 g, an effective intake of protein hydrolysates per mouse is 0.025 g when the minimum effective dose of protein hydrolysates is 1 g/kg. When this fraction is multiplied by the daily protein requirement for adults (80 g), the standard level of the minimum effective dose for adults is determined to be not less than 2 g/day. However, the minimum effective dose can be appropriately changed and determined in accordance with a person's body weight, age, sex, and fatigue level. Upon ingestion of the nutritional agent or the food of the present invention, the minimum amount required for a day may be ingested at once or may be ingested at multiple times in a divided manner.

The nutritional agent and the food of the present invention have effects whereby individuals can obviously recover from exhaustion by ingesting them when they are exhausted, and in addition, healthy individuals who are not exhausted can keep healthy or prevent exhaustion by ingesting them.

This specification includes part or all of the contents as disclosed in the specification and/or drawings of Japanese Patent Application No. 2004-048417, which is a priority document of the present application.

### Examples

Hereafter, the present invention will be described in more detail with reference to the following examples.

### (Reference example 1): A method for preparing peptides via enzymatic degradation (proteolysis using protease)

Germinated soybean cotyledon-derived protease D3 (the amount of which accounts for 0.5% of the protein weight of a substrate) or GSP (the amount of which accounts for 1% of the protein weight of a substrate) was added to 500 ml of a suspension (containing approximately 5% protein (NX6.25)) containing casein, wheat protein, or dried bonito flake powder. An enzymatic proteolysis reaction was carried out at pH 4.5 and 40°C in the case of D3 or at pH 8.0 and 45°C in the case of GSP. During enzymatic proteolysis reaction, a pH-stat was used to maintain the pH of the suspension at a constant level while adding 4M HCl or 4M NaOH thereto. 48 hours after enzymatic proteolysis reaction using D3 or 6 hours after enzymatic proteolysis reaction using GSP, the pH was adjusted to a roughly neutral level. Then, the suspension was heated at 80°C for 20 minutes for enzyme deactivation. Further, the suspension was subjected to centrifugation at 10,000 g for 5 minutes, resulting in precipitation of an insoluble substance therein. Thus, only a soluble fraction thereof was freeze dried so as to be subjected to a fatigue treatment test.

In addition, for comparison purposes, a protein hydrolysate was prepared using subtilisin and pepsin in accordance with the aforementioned method. In such cases, the amount of enzyme added accounted for 0.5% of the protein weight of a substrate. The enzymatic degradation reaction was carried out at a temperature of 40°C for 8 hours. At such time, the reaction pH was adjusted to pH 8.0 when using subtilisin or to pH 2.0 when using pepsin. (Example 1): Spontaneous motility recovery

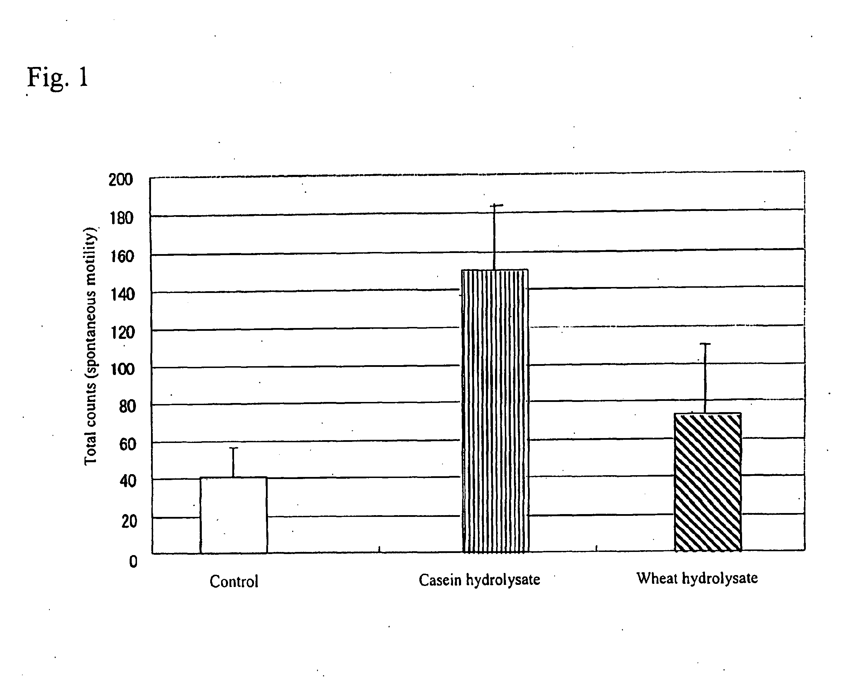

Groups of 5-week-old CDF-1 male mice (8 mice per group: n = 8) were placed on a treadmill so as to be subjected to forced walking exercise stress for 3 hours. Then, (1) casein hydrolysate by germinated soybean cotyledon-derived protease D3 and (2) wheat hydrolysate by germinated soybean cotyledon-derived protease D3 were each administered orally at 1 g (dry weight) /kg to a different group. Thereafter, the spontaneous motility of each group was measured for 60 minutes using a spontaneous motility measuring device equipped with an infrared sensor, followed by comparison with a control group (to which only deionized distilled water had been administered). Fig. 1 shows the results.

As shown in fig. 1, when administering a wheat protein hydrolysate and a casein hydrolysate, spontaneous motility was found to be improved. Particularly, when the casein protein hydrolysate was administered, spontaneous motility was significantly improved.

### (Example 2)

4 groups of 5-week-old CDF-1 male mice (8 mice per group: n = 8) were placed on a treadmill so as to be subjected to forced walking exercise stress for 3 hours. Then, (1) casein hydrolysate by germinated soybean cotyledon-derived protease D3, (2) casein hydrolysate by pepsin, (3) casein hydrolysate by subtilisin, and (4) casein hydrolysate by serine protease (GSP) were each administered orally at 1 g (dry weight) /kg to a different group. Thereafter, the spontaneous motility of each group was measured for 60 minutes using a spontaneous motility measuring device equipped with an infrared sensor, followed by comparison with a control group (to which only deionized distilled water had been administered).

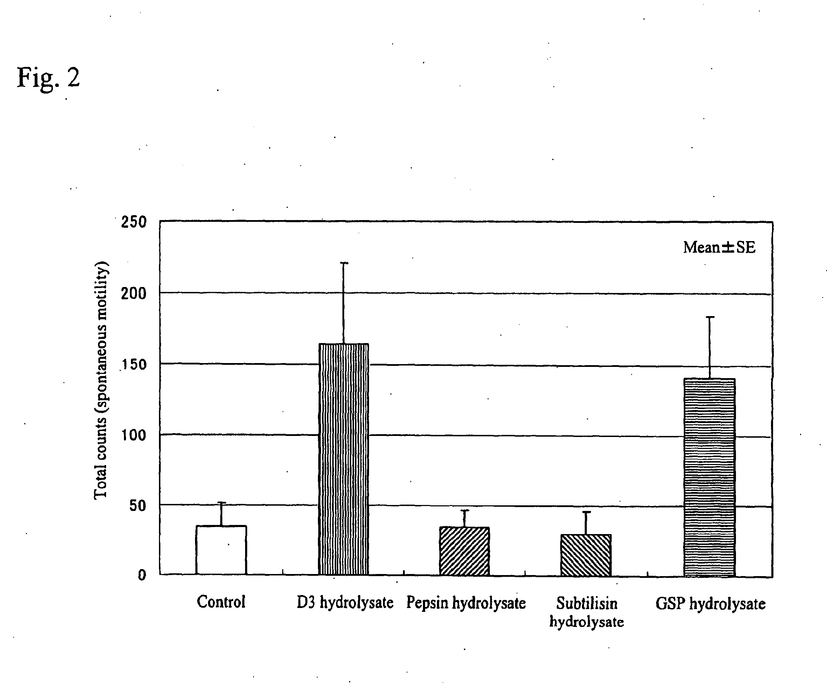

Fig. 2 shows the results. As is apparent from fig. 2, regarding the casein hydrolysate by germinated soybean cotyledon-derived protease D3 and the casein hydrolysate by serine protease (GSP), spontaneous motility was found to be significantly improved. However, spontaneous motility was not found to be improved in the group to which casein hydrolysate by pepsin had been administered or in the group to which casein hydrolysate by subtilisin had been administered.

### (Example 3)

3 groups of 5-week-old CDF-1 male mice (6 mice per group: n = 6) were placed on a treadmill so as to be subjected to forced walking exercise stress for 3 hours. Then, (1) casein protein, (2) an amino acid mixture (a mixture of amino acids that constitute casein protein: a mixture containing taurine, aspartic acid, threonine, serine, glutamic acid, proline, glycine, alanine, valine, methionine, isoleucine, leucine, tyrosine, phenylalanine, lysine, histidine, anserine, carnosine, and arginine), (3) peptides of dried bonito flakes degraded via germinated soybean cotyledon-derived protease D3, and (4) casein hydrolysate by germinated soybean cotyledon-derived protease D3 were each administered orally at 1 g (dry weight) /kg to a different group. Thereafter, the spontaneous motility of each group was measured for 60 minutes using a spontaneous motility measuring device equipped with an infrared sensor, followed by comparison with a control group (to which only deionized distilled water had been administered).

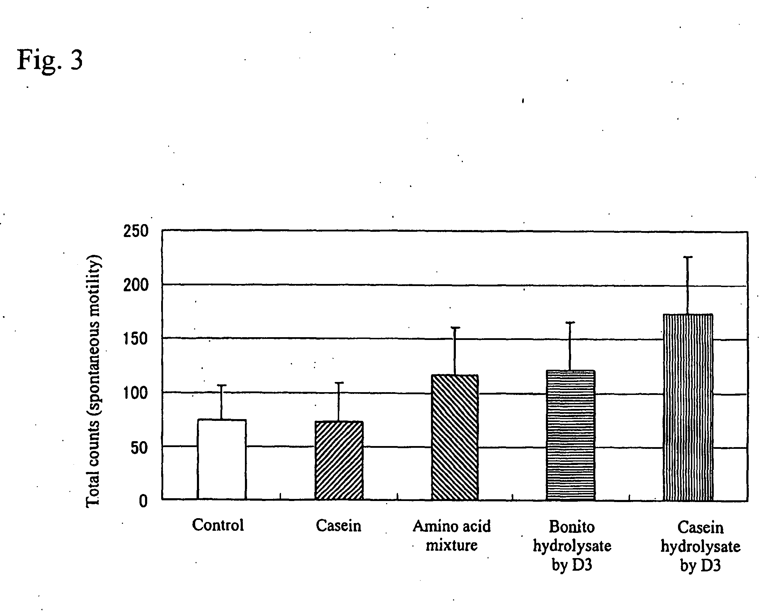

Fig. 3 shows the results. As is apparent from the figure, casein protein was not found to be effective. However, spontaneous motility was found to be significantly improved regarding the peptides of dried bonito flakes degraded via germinated soybean cotyledon-derived protease D3 and the casein hydrolysate by germinated soybean cotyledon-derived protease D3.

### (Example 4)

2 groups of 5-week-old CDF-1 male mice (6 mice per group: n = 8) were placed on a treadmill so as to be subjected to forced walking exercise stress for 3 hours. Then, (1) peptides 1 of casein degraded via germinated soybean cotyledon-derived protease D3 and (2) peptides 2 of casein degraded via germinated soybean cotyledon-derived protease D3 were each orally administered at doses of 0.5 g/kg and 1 g/kg, respectively, to a different group. Thereafter, the spontaneous motility of each group was measured for 60 minutes using a spontaneous motility measuring device equipped with an infrared sensor, followed by comparison with a control group (to which only deionized distilled water had been administered).

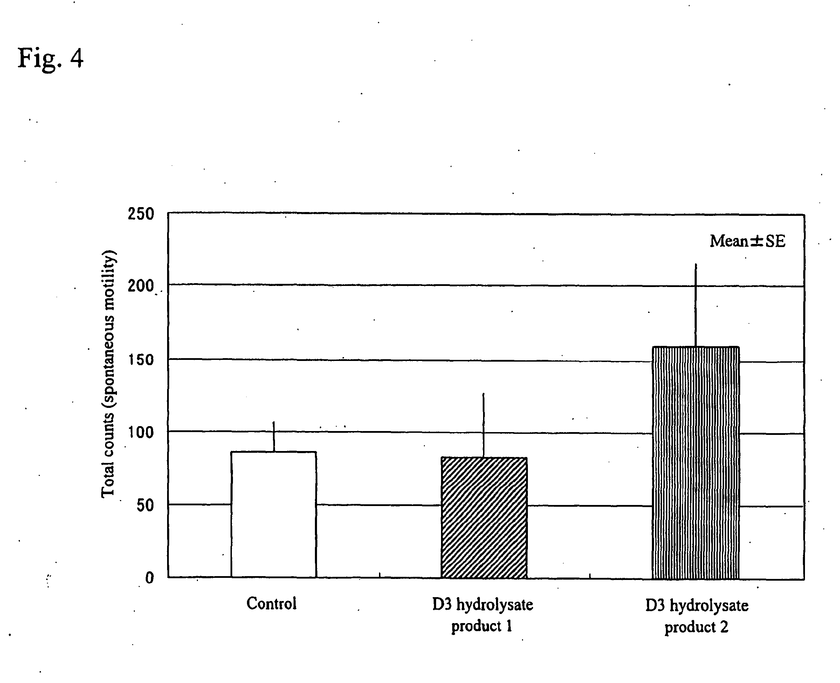

Fig. 4 shows the results. As is apparent from fig. 4, casein hydrolysate by germinated soybean cotyledon-derived protease D3 were found to be effective when administered at 1 g/kg.

### (Example 5)

2 groups of 5-week-old CDF-1 male mice (6 mice per group: n = 8) were placed on a treadmill so as to be subjected to forced walking exercise stress for 3 hours. Then, (1) peptides 1 of casein degraded via GSP, (2) peptides 2 of casein degraded by GSP, and (3) peptides 3 of casein degraded via GSP were each orally administered at doses of 0.5 g/kg, 1 g/kg, and 2 g/kg, respectively, to a different group. Thereafter, the spontaneous motility of each group was measured for 60 minutes using a spontaneous motility measuring device equipped with an infrared sensor, followed by comparison with control groups (a group to which only casein subjected to no enzymatic degradation had been administered and a group to which only deionized distilled water had been administered).

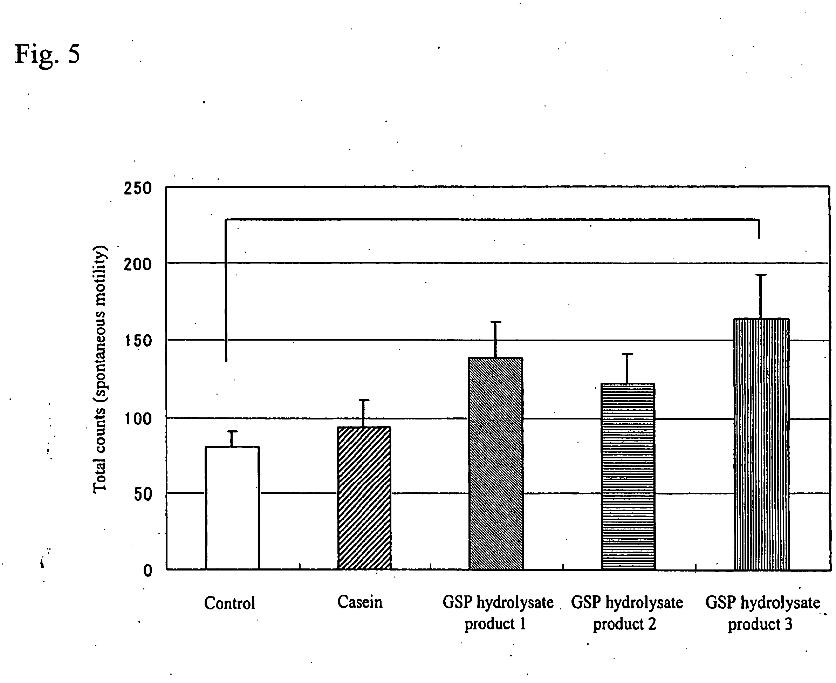

Fig. 5 shows the results. As is apparent from fig. 5, spontaneous motility was found to be significantly improved upon low-dose administration of casein hydrolysate by GSP at 0.5 g/kg.

### Industrial Applicability

The nutritional agent and the food described herein are each effective not only as a soothing agent or a food for treating fatigue that realizes quick recovery from exhaustion to a healthy state but also as a revitalizing agent or a revitalizing food whereby healthy individuals can prevent exhaustion or maintain a healthy state.

## Claims

1. Protein hydrolysates, hydrolysed by germinated soybean cotyledon-derived thiol protease and/or micro-organism derived serine protease, for use in a method of treating fatigue.

2. The protein hydrolysates of claim 1, in which the substrate protein is selected from the group consisting of casein, wheat protein, and dried bonito flakes.

3. The protein hydrolysates of claims 1 or 2 which are in the form of food.

4. Use of protein hydrolysates, hydrolysed by germinated soybean cotyledon-derived thiol protease and/or micro-organism derived serine protease, in the manufacture of a medicament for treating fatigue.

5. The use of claim 4, wherein the medicament is a food.

6. The use of claim 4 or claim 5, in which the substrate protein is selected from the group consisting of casein, wheat protein, and dried bonito flakes.

## Patentansprüche

1. Proteinhydrolysate, hydrolysiert durch von gekeimten Sojabohnenkotyledonen abgeleiteter Thiolprotease und/oder von Mikroorganismen abgeleiteter Serinprotease, zur Verwendung in einem Verfahren zur Behandlung von Erschöpfung.

2. Proteinhydrolysate nach Anspruch 1, worin das Substratprotein aus der Gruppe ausgewählt ist, die aus Casein, Weizenprotein und getrockneten Bonitoflocken besteht.

3. Proteinhydrolysate nach Anspruch 1 oder 2, die in der Form eines Nahrungsmittels sind.

4. Verwendung von Proteinhydrolysaten, hydrolysiert durch von gekeimten Sojabohnenkotyledonen abgeleiteter Thiolprotease und/oder von Mikroorganismen abgeleiteter Serinprotease, zur Herstellung eines Arzneimittels zur Behandlung von Erschöpfung.

5. Verwendung nach Anspruch 4, wobei das Arzneimittel ein Nahrungsmittel ist.

6. Verwendung nach Anspruch 4 oder 5, wobei das Substratprotein aus der Gruppe ausgewählt ist, die aus Casein, Weizenprotein und getrockneten Bonitoflocken besteht.

## Revendications

1. Hydrolysats de protéine, hydrolysés par une thiol-protéase dérivée de cotylédons de soja germés et/ou une sérine-protéase dérivée de micro-organismes, pour utilisation dans un procédé de traitement de la fatigue.

2. Hydrolysats de protéine selon la revendication 1, dans lesquels la protéine substrat est sélectionnée dans le groupe constitué de la caséine, de la protéine de blé et des écailles de bonite séchées.

3. Hydrolysats de protéine selon la revendication 1 ou 2, qui se présente sous la forme d'un aliment.

4. Utilisation d'hydrolysats de protéine, hydrolysés par une thiol-protéase dérivée de cotylédons de soja germés et/ou une sérine-protéase dérivée de micro-organismes, dans la préparation d'un médicament destiné au traitement de la fatigue.

5. Utilisation selon la revendication 4, où le médicament est un aliment.

6. Utilisation selon la revendication 4 ou la revendication 5, dans laquelle la protéine substrat est sélectionnée dans le groupe constitué de la caséine, de la protéine de blé et des écailles de bonite séchées.
